# EUROPEAN PATENT SPECIFICATION

(11) **EP 4 153 081 B1**
(45) Date of publication and mention of the grant of the patent: **27.03.2024**
(21) Application number: 21769298.7
(22) Date of filing: 20.08.2021
(51) Int. Cl.: A61B 18/14

(54) **DEVICE AND METHOD FOR DELIVERING ELECTROPORATION THERAPY**
VORRICHTUNG UND VERFAHREN ZUR VERABREICHUNG EINER ELEKTROPORATIONSTHERAPIE
DISPOSITIF ET PROCÉDÉ D'ADMINISTRATION D'UNE THÉRAPIE PAR ÉLECTROPORATION

(30) Priority: 26.08.2020 US 202063070706 P
(43) Date of publication of application: 29.03.2023
(73) Proprietor: St. Jude Medical, Cardiology Division, Inc., St. Paul MN 55117 (US)
(72) Inventor: TEGG, Troy, Elk River, Minnesota 55330 (US); SUTERMEISTER, Derek, Ham Lake, Minnesota 55304 (US)
(74) Representative: Alpspitz IP
(86) International application number: PCT/US2021/046811
(87) International publication number: WO 2022/046534

(56) References cited:
- EP-A1- 2 736 432
- WO-A1-2018/201037

## Description

### BACKGROUND

The present invention relates generally to medical devices and in particular to medical devices for delivering electroporation therapy.

Electroporation therapy involves the application of an electrical field to cell membranes to increase the permeability of the cell membrane. In some applications, electroporation is utilized to permanently damage or destroy targeted cells, which is referred to as irreversible electroporation (IRE) therapy. One of the benefits of IRE therapy is that it allows targeted cells to be damaged or ablated while leaving neighboring cells unaffected. IRE therapy is applicable to any procedure in which it is desirable to ablate tissue, including for example cancer therapy and/or cardiac ablation therapy to mitigate/cease certain arrhythmic conditions, including but limited to ectopic atrial tachycardia, atrial fibrillation, and atrial flutter. For example, EP 2 736 432 A1 discloses a mesh-overlayed ablation and mapping device.

In particular, IRE therapy stands to serve as an alternative to traditional cardiac ablation techniques. It is suspected that the primary cause of atrial arrhythmia is stray electrical signals within the left or right atrium of the heart. Traditional cardiac ablation therapy requires the delivery of ablative energy (e.g., radio frequency (rf) energy, lasers, etc.) to the cardiac tissue. The ablative energy - thermal energy - damages the cardiac tissue and disrupts the undesirable electrical pathways causing the arrhythmic condition.

IRE therapy allows for the targeted ablation of cardiac tissue to create the desired lesions - and therefore disruption of the undesirable electrical pathways - without the introduction of thermal energy that accompanies traditional ablative therapies.

### SUMMARY

The invention is defined in claim 1. Any methods disclosed hereinafter do not form part of the scope of the invention and are mentioned for illustrative purposes only. According to one aspect, an electroporation catheter includes a shaft having a proximal end and a distal end and an electroporation assembly located at the distal end of the shaft. The electroporation assembly may include at least a first electrode and a non-conductive portion, the non-conductive portion having a first end and a second end, wherein the first end is connected to the shaft and wherein the second end extends radially outward from the first end.

According to another aspect, a method of delivering electroporation therapy to a patient, wherein the method includes introducing a catheter into the patient, the catheter including a shaft having proximal end attached to a handle and a distal end that includes an electroporation assembly. The method may further includes navigating the electroporation assembly in an undeployed state to a desired tissue site, wherein the electroporation assembly includes at least a first electrode and a non-conductive portion attached on a first end to the shaft and a second, unattached end. At the desired tissue site, the electroporation assembly is deployed, wherein deployment of the electroporation assembly includes allowing the second, unattached end of the non-conductive portion to extend radially outward into contact with the desired tissue site. Electroporation pulses are then delivered to the first electrode.

### DESCRIPTION OF THE DRAWINGS

Figure 1 is a block diagram of a medical system for providing electroporation therapy according to some embodiments.
Figures 2A-2D are various views of an electroporation assembly including a non-conductive portion according to some embodiments.
Figure 3A is a side view of an electroporation assembly including a non-conductive portion in the geometry of a reverse umbrella sleeve according to some embodiments; and Figure 3B is a side view of the electroporation assembly including the non-conductive portion in a folded state according to some embodiments.
Figure 4 is a side view of an electroporation assembly including a non-conductive portion in the geometry of an umbrella according to some embodiments.
Figure 5 is a flowchart illustrating a method of delivering electroporation according to some embodiments.

### DETAILED DESCIRPTION

The disclosed invention is directed to a catheter that includes a shaft and an electroporation assembly. The shaft includes a proximal end and a distal end, wherein the electroporation assembly is connected to the distal end of the shaft. The electroporation assembly includes at least a first electrode and a non-conductive portion, wherein the non-conductive portion is connected on a first end to the shaft and extends radially outward from the shaft to a second end. The non-conductive portion prevents the formation of a low-impedance path between the first electrode and a second electrode - wherever located - that does not include the tissue to be treated. That is, deploying the non-conductive portion adjacent the tissue to be treated ensures that the electrical path formed between the electrodes delivering the pulses includes the tissue located adjacent the non-conductive portion.

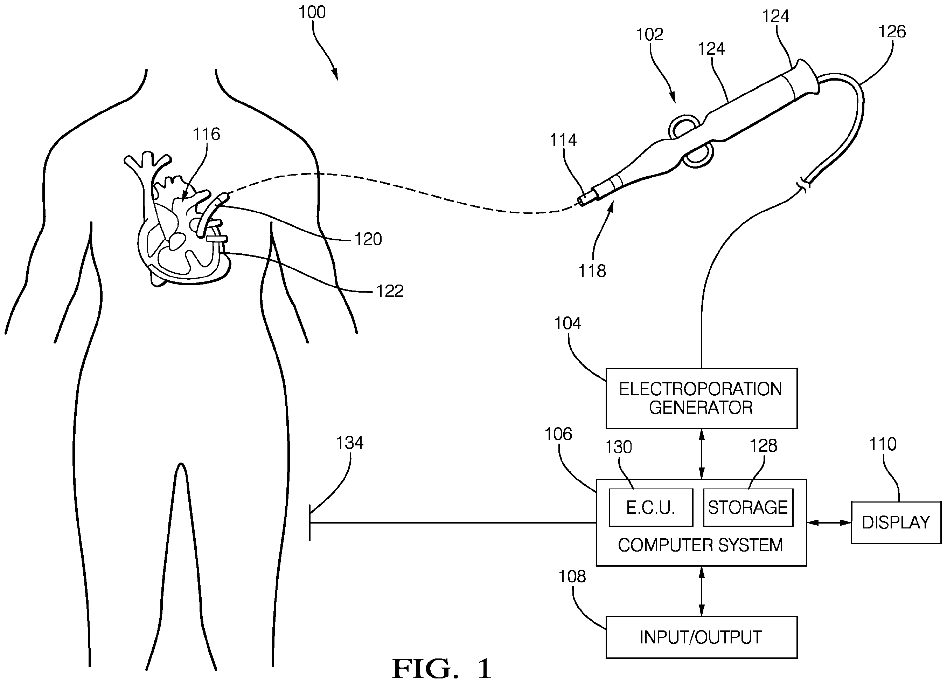

Figure 1 is a diagrammatic and block diagram view of a system 100 for providing electroporation therapy according to some embodiments. The system 100 includes a catheter 102, electroporation generator 104, computer system 106, input/output 108, and display 110. The catheter 102 includes a handle 112 and a shaft 114 having a distal end 116 and a proximal end 118. An electroporation assembly 120 is located at the distal end 116 of the shaft 114 and is configured to deliver electroporation therapy to adjacent tissue 122. In the embodiment shown in Figure 1, the electroporation assembly 120 is positioned to deliver electroporation therapy to cardiac tissue within the heart, but in other embodiments the distal end 116 of the catheter 102 - including the electroporation assembly 120 - may be positioned elsewhere within the body to deliver the desired electroporation therapy. Handle 112 is utilized to guide the distal end 116 of the shaft 114 to a desired location within the body. In some embodiments, handle 112 is utilized to passively or actively deploy the electroporation assembly 120 for delivery of the electroporation pulses. In some embodiments, the distal end 116 of the shaft includes one or more sensors (e.g., magnetic, optical, electrical, etc.) utilized for visualization and/or navigation of the distal end 116 to the desired location within the body.

Electroporation pulses delivered by the electroporation assembly 120 are generated by electroporation generator 104. In some embodiments, the magnitude, duration and number of pulses delivered may be modified by the electroporation generator 104. Additional information on generation and delivery of electroporation pulses is provided in U.S. Pat. Appl. 62/704,920, filed on June 3, 2020, titled "SYSTEM AND METHOD FOR IRREVERSIBLE ELECTROPORATION". Electroporation generator 104 is coupled to interface connector 124 of catheter 102 via cables 126. Electroporation pulses delivered to handle 102 are communicated via the shaft 114 to electroporation assembly 120. In addition, in some embodiments catheter 102 includes one or more sensors (e.g., magnetic, electric, optical, etc.) positioned along the shaft 114 for providing sensor inputs to the computer system 106. In some embodiments the same electrodes utilized to generate the electroporation pulses are also utilized to collect electrical signals (e.g., electrocardiogram signals, impedance signals, etc.) and are therefore provided to the computer system 106 via electroporation generator 104. In other embodiments, the sensed signals - whether electrical, magnetic, or optical - may be provided directly to computer system 106 (i.e., cables 126 may be coupled directly to computer system 106 rather than via electroporation generator 104).

In some embodiments, computer system 106 includes storage 128 capable of storing computer readable instructions and an electronic control unit (ECU 130) capable of executing computer readable instructions. In some embodiments, computer system 106 communicates bidirectionally with input/output 108 and further displays information via display 110. In some embodiments, computer system 106 controls generation and delivery of electroporation pulses by electroporation generator 104. This may include modifying one or more of the amplitude (i.e., intensity), duration, and number of pulses delivered by the electroporation generator 104.

In addition, computer system 106 may utilize the one or more sensed signals - whether electric, magnetic, or optical - as inputs to enable one or more functions including visualization, navigation, and/or mapping. For example, the one or more sensed signals may be utilized to visualize the location of the catheter within the patient's body as well as to aid in navigating the catheter through the patient's vasculature to the desired location within the patient's body. In some embodiments, electrical signals (e.g., electrocardiogram signals) sensed by electrodes associated with the electroporation assembly 120 may be utilized to map the propagation of electrical signals within the patient's heart or to verify that disruptive signals have been blocked following delivery of electroporation pulses. In some embodiments, a surface electrode 134 is utilized for receiving electrical signals and/or providing electrical signals to the body to be received by one or more electrodes located on the electroporation assembly 120. For example, an electrical signal generated at surface electrode 134 (or two or more surface electrodes) may be detected by one or more electrodes located at the distal end 116 of the catheter 102 within the patient's body. In other embodiments, surface electrode 134 may act as a return path for electrical signals delivered to the electroporation assembly 120 - including as a return for electroporation pulses delivered by electroporation generator 104 to the electroporation assembly 120.

In some embodiments one or more sensed signals are utilized to verify the position of the electroporation assembly 120 relative to the tissue to be electroporated. In particular, in some embodiments one or more sensed signals are utilized to verify that the electrical path between electrodes located on the electroporation assembly 120 includes the tissue selected to receive electroporation therapy. For example, in some embodiments a low impedance path between the electrodes located on the electroporation assembly 120 is utilized to detect a conductive path through the adjacent pool of blood - and which as a result does not include the higher impedance path through the tissue selected to receive electroporation treatment. In response, the electroporation assembly 120 may be repositioned and the electrical path between respective electrodes may be tested again.

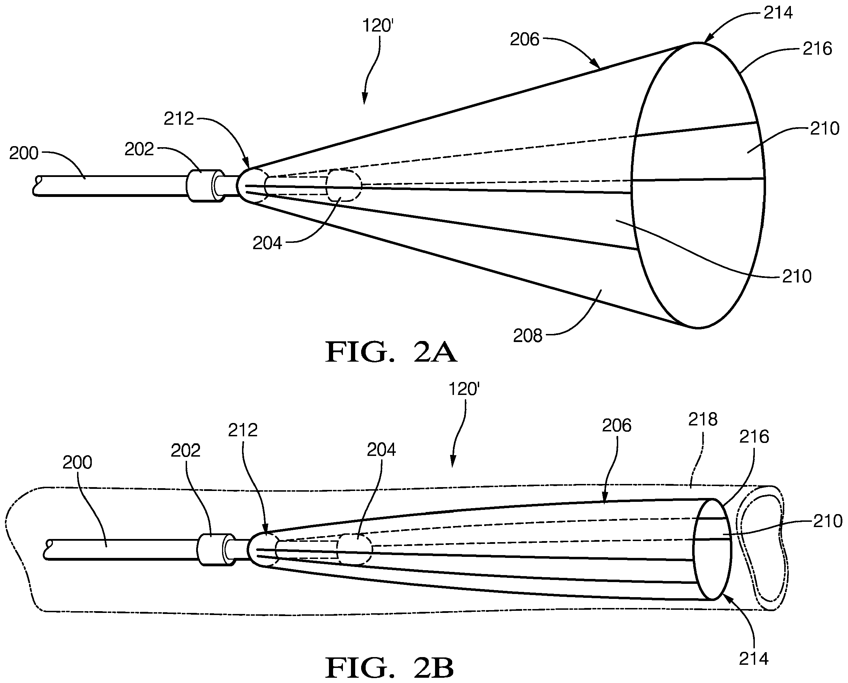

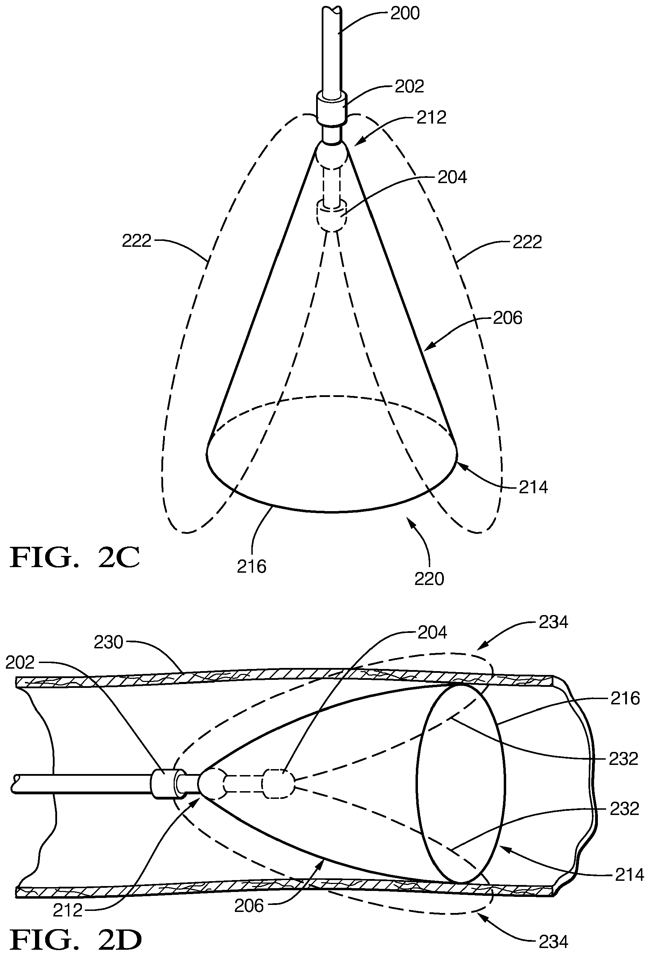

Referring now to Figures 2A-2D, an electroporation assembly 120' is illustrated according to some embodiments. In particular, Figure 2A illustrates the electroporation assembly 120' in a deployed state, Figure 2B illustrates the electroporation assembly 120' in an undeployed state, and Figures 2C and 2D illustrate various methods of positioning the electroporation assembly 120' to deliver electroporation therapy to the desired tissue.

In some embodiments, the electroporation assembly 120'is located at a distal end 116 of a shaft 114 (as shown in Figure 1). In some embodiments, the electroporation assembly 120' includes a shaft 200, a first electrode 202, a second electrode 204, and a non-conductive portion 206. In some embodiments, the shaft 200 may be an extension of shaft 114 (shown in Figure 1) or may be affixed to the distal end of shaft 114. In some embodiments, the first electrode 202 is located at a more proximal location (i.e., toward the handle 112, shown in Figure 1) and the second electrode 204 is located at a more distal location (i.e., away from the handle 112). In some embodiments, the first end 212 of the non-conductive portion 206 is affixed to the shaft 200 at a location between the first electrode 202 and the second electrode 204. The non-conductive portion 206 extends radially outward from the first end 212 to a second, unattached end 214. In some embodiments, the non-conductive portion 206 extends in a distal direction (i.e., away from the shaft 200). In other embodiments, described in more detail below, the non-conductive portion 206 may extend in a proximal direction from first end 212 to the second end 214. The second end 214 of the non-conductive portion includes an edge 216 that defines a perimeter of the non-conductive portion 206. In embodiments in which the non-conductive portion 206 extends in a distal direction from first end 212 to the second end 214, the non-conductive portion 206 extends around or surrounds the second electrode 204, as indicated by the dashed lines utilized to display a portion of shaft 200 and the second electrode 204. In some embodiments, the geometry of non-conductive portion is a hollow cone in which the first end 212 represents the apex of the cone and the second end 214 represents the base of the cone. In some embodiments the edge 216 provided at the second end 214 is circular, but in other embodiments may be defined by other geometries (e.g., oval).

Electroporation therapy includes the delivery or one or more electrical pulses between a first and second electrode. For example, in the embodiment shown in Figures 2A and 2B the electroporation pulses may be delivered between the first electrode 202 and the second electrode 204. In some embodiments, one of the electrodes utilized to deliver the electroporation pulse may be located on the non-conductive portion 206 rather than on the shaft 200. For example, in some embodiments rather than locate first electrode 202 on the shaft 200, the first electrode 202 is located on an outer surface of the non-conductive portion 206. In other embodiments, the electroporation pulse may be delivered between a first electrode (either electrode 202 or electrode 204) and another electrode located either on a separate catheter, separate medical device, or elsewhere on the patient (e.g., skin electrode). In some embodiments, electrode 204 located within the space defined by the non-conductive portion 206 is utilized to deliver the electroporation pulse if the second electrode is located separated from the electroporation assembly. For the sake of simplicity, delivery of electroporation pulses is described with respect to delivery between the first electrode 202 and the second electrode 204. The electrical path between the first electrode 202 and the second electrode 204 is always defined by the path of least resistance (i.e., lowest impedance). Because blood pools present a lower impedance than tissue, the electrical path between the first electrode 202 and the second electrode 204 - without the presence of non-conductive portion 206 - may bypass the tissue to be treated. The purpose of non-conductive portion 206 is to ensure that the conductive path formed between the first electrode 202 and the second electrode 204 is through the tissue to be treated.

Figure 2A illustrates the non-conductive portion 206 in a deployed state utilized to deliver electroporation therapy and Figure 2B illustrates the non-conductive portion 206 in an undeployed state utilized to transport the electroporation assembly 120' to a desired location within the patient's body. In the undeployed state the non-conductive portion 206 is compressed or deformed to provide a smaller circumference and/or surface area (when viewed from the end of the electroporation assembly 120') as compared with the deployed state. For example, in the embodiment shown in Figure 2B the non-conductive portion is compressed/folded to fit within a sheath 218. Upon reaching the desired location, the non-conductive portion 206 is deployed through either passive or active means. For example, passive deployment may be provided by removing the sheath 218 from the portion of the electroporation assembly 120' that covers the non-conductive portion 206, allowing the non-conductive portion 206 to expand. In some embodiments, the non-conductive portion 206 may comprise memory shape material utilized to cause the non-conductive portion 206 to expand from the undeployed state shown in Figure 2B to the deployed state shown in Figure 2A. In other embodiments, the non-conductive portion 206 is actively deployed via a mechanism (not shown) utilized to expand the non-conductive portion 206 from the undeployed state to the deployed state. For example, a wire (not shown) connected between the handle 112 and non-conductive portion 206 may be utilized to deploy the non-conductive portion.

In some embodiments, the non-conductive portion 206 is comprised of a uniform non-conductive material (i.e., insulative material). Depending on the application the non-conductive portion 206 may be comprised of compliant or non-compliant materials. For example, in some embodiments the non-conductive portion 206 may be fabricated using one or more of silicone, polyurethane, rubber, and/or a non-conductive polymer, each of which is compliant and non-conductive. One benefit of utilizing a compliant material is that it allows the non-conductive portion 206 to be deformed/compressed in an undeployed state to allow the non-conductive portion to fit within a sheath 218 as shown in Figure 2B for delivery to the desired location. In addition, in some embodiments utilization of a compliant material allows the non-conductive portion 206 to conform to the geometry of surrounding tissue during electroporation treatment. In some embodiments, such as that shown in Figures 2A, 2B, the non-conductive portion 206 may be comprised of a two or more different materials. For example, in some embodiments, the non-conductive portion 206 includes a first non-conductive material 208 and a memory shape non-conductive material 210 (e.g., nitinol). In the embodiment shown in Figures 2A and 2B the memory shape material 210 defines the geometry of the non-conductive portion 206 in the deployed state (e.g., cone shape shown in Figure 2A) while allowing the non-conductive portion 206 to be compressed or deformed as shown in Figure 2B.

Figures 2C and 2D illustrates positioning of the electroporation assembly 120' relative to tissue to be treated according to various embodiments. In the embodiment shown in Figure 2C, the edge 216 of non-conductive portion 206 is placed against the tissue 220 to be treated. The non-conductive path formed between the first electrode 202 and the second electrode 204 - illustrated by lines 222 - includes the tissue 220 located adjacent to the edge 216 of the non-conductive portion 206. In the embodiment shown in Figure 2D the electroporation assembly 120' is placed within a tubular structure 230 such as a pulmonary vein. The non-conductive portion 206 is expanded such that an outer circumferential surface of the non-conductive portion 206 is brought into contact with the interior surface of the tubular structure 230 (e.g., pulmonary vein). The non-conductive path formed between the first electrode 202 and the second electrode 204 - illustrated by lines 232 - includes the tissue 234 located adjacent the portion of non-conductive portion 206 brought into contact with the tubular structure 230. One of the benefits of the electroporation assembly 120' is that it is capable of being utilized to provide electroporation therapy to relatively planar tissue sites (as shown in Figure 2C) as well as tubular tissue sites (as shown in Figure 2D).

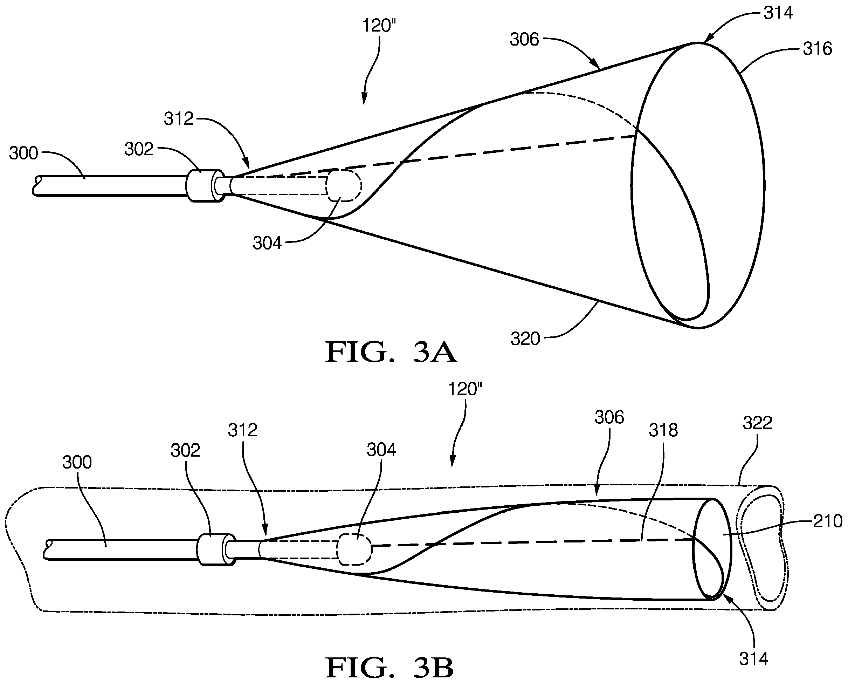

Referring now to Figures 3A-3B, an electroporation assembly 120" is illustrated according to some embodiments. In particular, Figure 3A illustrates the electroporation assembly 120" in a deployed state and Figure 3B illustrates the electroporation assembly 120" in an undeployed state.

As described above, in some embodiments the electroporation assembly 120" is located at a distal end 116 of a shaft 114 (as shown in Figure 1). In some embodiments, the electroporation assembly 120" includes a shaft 300, a first electrode 302, a second electrode 304, and a non-conductive portion 306. As discussed above, the shaft 300 may be an extension of shaft 114 (shown in Figure 1) or may be affixed to the distal end of shaft 114. In some embodiments, the first electrode 302 is located at a more proximal location (i.e., toward the handle 112, shown in Figure 1) and the second electrode 304 is located at a more distal location (i.e., away from the handle 112). In some embodiments, the non-conductive portion 306 has a first end 312 and a second end 314 extending radially outward from the first end 312. In some embodiments, the first end 312 of the non-conductive portion 306 is affixed to the shaft 300 at a location between the first electrode 302 and the second electrode 304. In some embodiments, the non-conductive portion extends in a distal direction from the first end 312 to the second end 314 (for example, as shown in Figures 3A and 3B). In other embodiments, the non-conductive portion 306 may extend in a proximal direction from the first end 312 to the second end 314. In some embodiments, the non-conductive portion 306 is a discontinuous sleeve having a first edge 318 and a second edge 320. In some embodiments, the first edge 318 is located on an inner part of the sleeve. The geometry of the non-conductive portion can be compressed by rolling the first edge 318 within the sleeve. In this way, the sleeve-like geometry of the non-conductive portion 306 shown in Figures 3A and 3B can be rolled up into a compressed state (i.e., undeployed state) for transport. In some embodiments, the non-conductive portion 306 in the undeployed or rolled-up state is configured to fit within a sheath 322 as shown in Figure 3B. In some embodiments, the sleeve of the non-conductive portion 306 may be deployed by removing the sheath 322 constraining the non-conductive portion, allowing the non-conductive portion 306 to unroll into a deployed state.

The geometry of the electroporation assembly 120" shown in Figures 3A and 3B allows the electroporation assembly to be utilized to deliver electroporation therapy to planar tissue sites (for example, as shown in Figure 2C) and/or to tubular tissue sites (for example, as shown in Figure 2D). In some embodiments, the sleeve-like non-conductive portion 306 may be fabricated using one or more of silicone, polyurethane, rubber, and/or a non-conductive polymer. The sleeve-like non-conductive portion 306 may likewise be compliant or non-compliant. One benefit of utilizing a compliant material is that it allows the non-conductive portion 306 to conform to the geometry of surrounding tissue during electroporation treatment. For example, a compliant non-conductive portion 306 may be provide benefits in conforming with semi-planar tissue as well as non-circular tubular tissue.

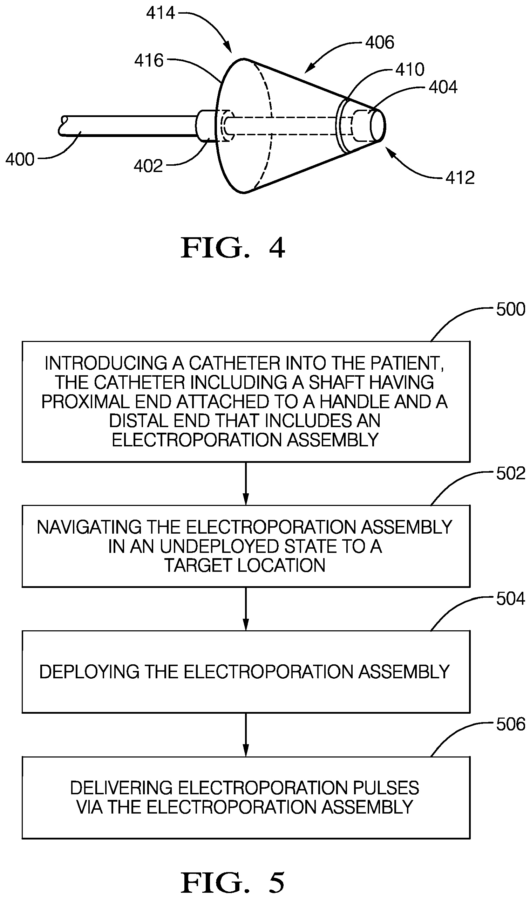

Referring now to Figure 4, an electroporation assembly 120‴ is illustrated according to some embodiments. As described above, in some embodiments the electroporation assembly 120‴ is located at a distal end 116 of a catheter 102 (as shown in Figure 1). In some embodiments, the electroporation assembly 120‴ includes a shaft 400, a plurality of possible electrode locations as shown by electrodes 402, 404, and 410, a non-conductive portion 406. As discussed above, the shaft 400 may be an extension of shaft 114 (shown in Figure 1) or may be affixed to the distal end of shaft 114. In the embodiment shown in Figure 4, the non-conductive portion 406 includes a first end 412 and a second end 414 extending radially outward from the first end 412. In the embodiment shown in Figure 4 the second end 414 extends in a proximal direction - rather than a distal direction - from the first end 412. The geometry provided in this embodiment may be referred to as an umbrella geometry based on the shape of the non-conductive portion relative to the shaft 400. In this embodiment, the first end 412 of the non-conductive portion 406 is coupled to the shaft at a location distal to both the first electrode 402 and the second electrode 404. However, as described in more detail below in other embodiments the first end 412 of the non-conductive portion 406 may be coupled to other locations along the shaft relative to first electrode 402 and second electrode 404, depending in particular on the location of the third electrode 410 on either an inner surface or outer surface of the non-conductive portion 406.

In some embodiments, electroporation pulses are delivered between electrode 402 and electrode 410 or between electrode 404 and electrode 410. The conductive path formed between either of the electrodes 402, 404 and the electrode 410 located on an outer surface 408 of non-conductive portion 406 is forced by the presence of non-conductive portion 406 to include tissue located adjacent to the non-conductive portion 406. In other embodiments, based on wherein the first end 412 of the non-conductive portion 406 is connected to the shaft 400 relative to the first electrode 402 and/or second electrode 404, the third electrode 410 may be located on an inner surface of the non-conductive portion 406. The key is that the conductive path formed between the two electrodes selected to deliver the electroporation pulses is forced by the presence of the non-conductive portion 406 to include the tissue located adjacent the non-conductive portion 406.

As discussed above, the non-conductive portion 406 may be compliant or non-compliant. In some embodiments, a benefit of utilizing a compliant material is the ability to deform/compress the non-conductive portion 406 to a smaller diameter that allows the non-conductive portion 406 to fit within a sheath (not shown) in a manner similar to that shown in Figure 2B. In some embodiments, the electroporation assembly is transported to the desired location in the undeployed state. Upon reaching the desired location, the electrode assembly 120‴ is either passively or actively deployed as shown in Figure 4. For example, in some embodiments the non-conductive portion is deployed by pulling back on a sheath surrounding the electroporation assembly 120‴ to expose the non-conductive portion 406. In some embodiments, the non-conductive portion 406 may include memory shape material to provide a desired shape in the deployed state.

Figure 5 is a flowchart illustrating a method of delivering electroporation according to some embodiments. At step 500, a catheter is introduced into the patient, wherein the catheter includes a shaft having proximal end attached to a handle and a distal end that includes an electroporation assembly.

At step 502, the electroporation assembly in an undeployed state is navigated to a desired tissue site within the patient. For example, Figures 2B and 3B illustrate examples of the electroporation assembly in an undeployed or compressed state in which the non-conductive portion is compressed or deformed to a smaller radius. In some embodiments, one or more sensors included as part of the electroporation assembly may be utilized to aid in navigating the electroporation assembly to the desired tissue site. For example, one or more of the electrodes may be utilized for visualization and/or navigation of the electroporation assembly to the desired location within the body.

At step 504, having reach the target tissue site, the electroporation assembly is deployed. Examples of the electroporation assembly in the deployed state are shown in Figures 2A, 2C, 2D, 3A, and 4. In the deployed state, the non-conductive portion prevents the formation of a low-impedance path between the electrodes utilized to deliver the electroporation therapy.

At step 506, electroporation pulses are delivered via the electroporation assembly. For example, in some embodiments the electroporation assembly includes first and second electrode utilized to deliver the electroporation pulses, wherein the non-conductive portion ensures that the conductive path between the electrodes includes the tissue to be treated. In other embodiments, the electroporation assembly may include only a single electrode, with the other electrode located separately from the electroporation assembly.

While the invention has been described with reference to an exemplary embodiment(s), it will be understood by those skilled in the art that various changes may be made and equivalents may be substituted for elements thereof without departing from the scope of the invention. In addition, many modifications may be made to adapt a particular situation or material to the teachings of the invention without departing from the essential scope thereof. Therefore, it is intended that the invention not be limited to the particular embodiment(s) disclosed, but that the invention will include all embodiments falling within the scope of the appended claims.

### Discussion of Possible Embodiments

The following are non-exclusive descriptions of possible embodiments of the present invention.

According to one aspect, an electroporation catheter includes a shaft having a proximal end and a distal end and an electroporation assembly located at the distal end of the shaft. The electroporation assembly includes at least a first electrode and a non-conductive portion, the non-conductive portion having a first end and a second end, wherein the first end is connected to the shaft and wherein the second end extends radially outward from the first end.

The device of the preceding paragraph can optionally include, additionally and/or alternatively any one or more of the following features, configurations, and/or additional components.

For example, in some embodiments, the non-conductive portion may be cone-shaped having an apex at the first end connected to the shaft.

In some embodiments, the non-conductive portion may extend away from the shaft in a distal direction from the first end to the second end.

In some embodiments, the electroporation assembly may further includes a second electrode, wherein the second electrode may be proximally located along the shaft relative to the first electrode and wherein the first end of the non-conductive portion may be connected to the shaft between the first electrode and the second electrode.

In some embodiments, the non-conductive portion may extend in a proximal direction toward the shaft from the first end to the second end.

In some embodiments, the electroporation assembly may further include a second electrode located on an outer surface of the non-conductive portion, wherein the second electrode may be located proximal to the first end of the non-conductive portion.

In some embodiments, the non-conductive portion may be a sleeve.

In some embodiments, the non-conductive portion may be movable between an undeployed state and a deployed state, wherein a radius of the non-conductive portion is greater in the deployed state.

In some embodiments, the non-conductive portion may be passively deployed via removal of a sheath located on an outer periphery of the non-conductive portion.

In some embodiments, the non-conductive portion may be actively deployed via a mechanism having a first end coupled to the electroporation assembly and a second end connected to a handle located on the proximal end of the shaft.

In some embodiments, the non-conductive portion may be comprised, at least in part, of a compliant, non-conductive material.

In some embodiments, the non-conductive portion may be comprised, at least in part, of a memory shape material.

In some embodiments, the compliant, non-conductive material may be comprised of one or more of silicone, polyurethane, rubber, and/or a non-conductive polymer.

In some embodiments, the non-conductive portion may be comprised of a non-compliant, non-conductive material.

According to another aspect, a method of delivering electroporation therapy to a patient, wherein the method includes introducing a catheter into the patient, the catheter including a shaft having proximal end attached to a handle and a distal end that includes an electroporation assembly. The method may further includes navigating the electroporation assembly in an undeployed state to a desired tissue site, wherein the electroporation assembly includes at least a first electrode and a non-conductive portion attached on a first end to the shaft and a second, unattached end. At the desired tissue site, the electroporation assembly is deployed, wherein deployment of the electroporation assembly includes allowing the second, unattached end of the non-conductive portion to extend radially outward into contact with the desired tissue site. Electroporation pulses are then delivered to the first electrode.

The method of the preceding paragraph can optionally include, additionally and/or alternatively any one or more of the following steps.

For example, in some embodiments, the electroporation assembly may be passively deployed by removing a sheath surrounding the non-conductive portion of the electroporation assembly.

In some embodiments, the electroporation assembly may be actively deployed by activating a mechanism in the handle to cause the second end of non-conductive portion to expand radially outward.

In some embodiments, an edge associated with the second end of the non-conductive portion may be brought into contact with the tissue to be treated, wherein the tissue to be treated is approximately planar.

In some embodiments, an outer surface of the non-conductive portion may be brought into contact with the tissue to be treated, wherein the tissue to be treated is tubular.

In some embodiments, wherein the electroporation therapy may be an irreversible electroporation therapy.

## Claims

1. An electroporation catheter (102) comprising:
a shaft (114, 200, 300, 400) having a proximal end (118) and a distal end (116); and
an electroporation assembly (120, 120', 120") located at the distal end (116) of the shaft (114,200,300, 400), the electroporation assembly (120, 120', 120") having at least a first electrode (204, 304, 404), a second electrode (202, 302) proximally located relative to the first electrode (204, 304, 404), said electroportaion catheter further comprising and a non-conductive portion (206, 306, 406), the non-conductive portion (206, 306, 406) having a first end (212, 312, 412) and a second end (214, 314, 414), wherein the first end (212, 312, 412) is connected to the shaft (114, 200, 300, 400) and wherein the second end (214, 314, 414) extends radially outward from the first end (212, 312, 412), **characterised in that** said second electrode is located along the shaft (114, 200, 300, 400).

2. The electroporation catheter (102) of claim 1, wherein the non-conductive portion (206, 306, 406) is cone-shaped having an apex at the first end (212, 312, 412) connected to the shaft (114).

3. The electroporation catheter (102) of claim 2, wherein the non-conductive portion (206, 306, 406) extends away from the shaft (114, 200, 300, 400) in a distal direction from the first end (212, 312, 412) to the second end (214, 314, 414).

4. The electroporation catheter (102) of claim 3, wherein the first end (212, 312) of the non-conductive portion (206, 306) is connected to the shaft (114, 200, 300, 400) between the first electrode (204, 304), and the second electrode (202, 302).

5. The electroporation catheter (102) of claim 2, wherein the non-conductive portion (406) extends in a proximal direction toward the shaft (114, 200, 300, 400) from the first end (412) to the second end (414).

6. The electroporation catheter (102) of claim 5, wherein the electroporation assembly (120, 120', 120") further includes a third electrode (410) located on an outer surface of the non-conductive portion (406), wherein the second electrode is located proximal to the first end (412) of the non-conductive portion (406).

7. The electroporation catheter (102) of any one of claims 1 to 6, wherein the non-conductive portion is a sleeve.

8. The electroporation catheter (102) of any one of claims 1 to 7, wherein the non-conductive portion (206, 306, 406) is movable between an undeployed state and a deployed state, wherein a radius of the non-conductive portion (206, 306, 406) is greater in the deployed state.

9. The electroporation catheter (102) of claim 8, wherein the non-conductive portion (206, 306) is passively deployed via removal of a sheath (218, 322) located on an outer periphery of the non-conductive portion (206, 306).

10. The electroporation catheter (102) of claim 8, wherein the non-conductive portion (206) is actively deployed via a mechanism having a first end coupled to the electroporation assembly (120) and a second end connected to a handle (112) located on the proximal end (118) of the shaft (114, 200, 300, 400).

11. The electroporation catheter (102) of claim 1, wherein the non-conductive portion (206, 306, 406) comprises a compliant, non-conductive material.

12. The electroporation catheter (102) of claim 11, wherein the non-conductive portion (206, 306, 406) further comprises a memory shape material.

13. The electroporation catheter (102) of claim 12, wherein the compliant, non-conductive material comprises one or more silicone, polyurethane, rubber, and/or a non-conductive polymer.

14. The electroporation catheter (102) of claim 1, wherein the non-conductive portion (206, 306, 406) comprises a non-compliant, non-conductive material.

## Patentansprüche

1. Elektroporationskatheter (102), enthaltend:
einen Schaft (114, 200, 300, 400) mit einem proximalen Ende (118) und einem distalen Ende (116); und
einer Elektroporationsbaugruppe (120, 120', 120"), die sich an dem distalen Ende (116) des Schafts (114, 200, 300, 400) befindet, wobei die Elektroporationsbaugruppe (120, 120', 120") wenigstens aufweist
eine erste Elektrode (204, 304, 404),
eine zweite Elektrode (202, 302), die sich proximal relativ zu der ersten Elektrode (204, 304, 404) befindet,
wobei der Elektroporationskatheter ferner enthält
und einen nicht-leitenden Bereich (206, 306, 406), wobei der nicht-leitende Bereich (206, 306, 406) ein erstes Ende (212, 312, 412) und ein zweites Ende (214, 314, 414) aufweist,
wobei das erste Ende (212, 312, 412) mit dem Schaft (114, 200, 300, 400) verbunden ist und wobei das zweite Ende (214, 314, 414) sich radial von dem ersten Ende (212, 312, 412) nach außen erstreckt, **dadurch gekennzeichnet, dass** die zweite Elektrode sich entlang des Schafts (114, 200, 300, 400) befindet.

2. Elektroporationskatheter (102) nach Anspruch 1, wobei der nicht-leitende Bereich (206, 306, 406) kegelförmig ist mit einer Spitze an dem ersten Ende (212, 312, 412), das mit dem Schaft (114) verbunden ist.

3. Elektroporationskatheter (102) nach Anspruch 2, wobei der nicht-leitende Bereich (206, 306, 406) sich von dem Schaft (114, 200, 300, 400) in einer distalen Richtung von dem ersten Ende (212, 312, 412) zu dem zweiten Ende (214, 314, 414) weg erstreckt.

4. Elektroporationskatheter (102) nach Anspruch 3, wobei das erste Ende (212, 312) des nicht-leitenden Bereichs (206, 306) zwischen der ersten Elektrode (204, 304) und der zweiten Elektrode (202, 302) mit dem Schaft (114, 200, 300, 400) verbunden ist.

5. Elektroporationskatheter (102) nach Anspruch 2, wobei der nicht-leitende Bereich (406) sich in einer proximalen Richtung von dem ersten Ende (412) zu dem zweiten Ende (414) zu dem Schaft (114, 200, 300, 400) erstreckt.

6. Elektroporationskatheter (102) nach Anspruch 5, wobei die Elektroporationsbaugruppe (120, 120', 120") ferner eine dritte Elektrode (410) aufweist, die sich auf einer Außenfläche des nicht-leitenden Bereichs (406) befindet, wobei die zweite Elektrode sich proximal zu dem ersten Ende (412) des nicht-leitenden Bereichs (406) befindet.

7. Elektroporationskatheter (102) nach einem der Ansprüche 1 bis 6, wobei der nicht-leitende Bereich eine Hülse ist.

8. Elektroporationskatheter (102) nach einem der Ansprüche 1 bis 7, wobei der nicht-leitende Bereich (206, 306, 406) zwischen einem nicht entfalteten Zustand und einem entfalteten Zustand beweglich ist, wobei ein Radius des nicht-leitenden Bereichs (206, 306, 406) in dem entfalteten Zustand größer ist.

9. Elektroporationskatheter (102) nach Anspruch 8, wobei der nicht-leitende Bereich (206, 306) passiv entfaltet wird über ein Entfernen einer Umhüllung (218, 322), die sich auf einem Außenumfang des nicht-leitenden Bereichs (206, 306) befindet.

10. Elektroporationskatheter (102) nach Anspruch 8, wobei der nicht-leitende Bereich (206) aktiv entfaltet wird über einen Mechanismus, der ein erstes Ende gekoppelt mit der Elektroporationsbaugruppe (120) und ein zweites Ende gekoppelt mit einem Handgriff (112), der sich auf dem proximalen Ende (118) des Schafts (114, 200, 300, 400) befindet, aufweist.

11. Elektroporationskatheter (102) nach Anspruch 1, wobei der nicht-leitende Bereich (206, 306, 406) ein nachgiebiges, nicht-leitendes Material aufweist.

12. Elektroporationskatheter (102) nach Anspruch 11, wobei der nicht-leitende Bereich (206, 306, 406) ferner ein Formgedächtnismaterial aufweist.

13. Elektroporationskatheter (102) nach Anspruch 12, wobei das nachgiebige, nicht-leitende Material eines oder mehrere aus Silikon, Polyurethan, Gummi und/oder einem nicht-leitenden Polymer aufweist.

14. Elektroporationskatheter (102) nach Anspruch 1, wobei der nicht-leitende Bereich (206, 306, 406) ein nicht nachgiebiges, nicht-leitendes Material aufweist.

## Revendications

1. Cathéter d'électroporation (102) comprenant :
une tige (114, 200, 300, 400) ayant une extrémité proximale (118) et une extrémité distale (116) ; et
un ensemble d'électroporation (120, 120', 120") situé à l'extrémité distale (116) de la tige (114, 200, 300, 400), l'ensemble d'électroporation (120, 120', 120") ayant au moins une première électrode (204, 304, 404), une seconde électrode (202, 302) située de manière proximale par rapport à la première électrode (204, 304, 404),
ledit cathéter d'électroporation comprenant en outre
et une partie non conductrice (206, 306, 406), la partie non conductrice (206, 306, 406) ayant une première extrémité (212, 312, 412) et une seconde extrémité (214, 314, 414), dans lequel la première extrémité (212, 312, 412) est reliée à la tige (114, 200, 300, 400) et dans lequel la seconde extrémité (214, 314, 414) s'étend radialement vers l'extérieur à partir de la première extrémité (212, 312, 412), **caractérisé en ce que** ladite seconde électrode est située le long de la tige (114, 200, 300, 400).

2. Cathéter d'électroporation (102) de la revendication 1, dans lequel la partie non conductrice (206, 306, 406) est en forme de cône ayant un apex à la première extrémité (212, 312, 412) reliée à la tige (114).

3. Cathéter d'électroporation (102) de la revendication 2, dans lequel la partie non conductrice (206, 306, 406) s'étend à l'écart de la tige (114, 200, 300, 400) dans une direction distale de la première extrémité (212, 312, 412) à la seconde extrémité (214, 314, 414).

4. Cathéter d'électroporation (102) de la revendication 3, dans lequel la première extrémité (212, 312) de la partie non conductrice (206, 306) est connectée à la tige (114, 200, 300, 400) entre la première électrode (204, 304) et la seconde électrode (202, 302).

5. Cathéter d'électroporation (102) de la revendication 2, dans lequel la partie non conductrice (406) s'étend dans une direction proximale vers la tige (114, 200, 300, 400) de la première extrémité (412) à la seconde extrémité (414).

6. Cathéter d'électroporation (102) de la revendication 5, dans lequel l'ensemble d'électroporation (120, 120', 120") comprend en outre une troisième électrode (410) située sur une surface extérieure de la partie non conductrice (406), dans laquelle la seconde électrode est située à proximité de la première extrémité (412) de la partie non conductrice (406).

7. Cathéter d'électroporation (102) de l'une quelconque des revendications 1 à 6, dans lequel la partie non conductrice est un manchon.

8. Cathéter d'électroporation (102) de l'une quelconque des revendications 1 à 7, dans lequel la partie non conductrice (206, 306, 406) est mobile entre un état non déployé et un état déployé, dans lequel un rayon de la partie non conductrice (206, 306, 406) est plus grand dans l'état déployé.

9. Cathéter d'électroporation (102) de la revendication 8, dans lequel la partie non conductrice (206, 306) est déployée passivement par le retrait d'une gaine (218, 322) située sur une périphérie extérieure de la partie non conductrice (206, 306).

10. Cathéter d'électroporation (102) de la revendication 8, dans lequel la partie non conductrice (206) est activement déployée via un mécanisme ayant une première extrémité couplée à l'ensemble d'électroporation (120) et une seconde extrémité connectée à une poignée (112) située sur l'extrémité proximale (118) de la tige (114, 200, 300, 400).

11. Cathéter d'électroporation (102) de la revendication 1, dans lequel la partie non conductrice (206, 306, 406) comprend un matériau conforme et non conducteur.

12. Cathéter d'électroporation (102) de la revendication 11, dans lequel la partie non conductrice (206, 306, 406) comprend en outre un matériau à mémoire de forme.

13. Cathéter d'électroporation (102) de la revendication 12, dans lequel le matériau conforme et non conducteur comprend un ou plusieurs silicones, polyuréthanes, caoutchoucs et/ou un polymère non conducteur.

14. Cathéter d'électroporation (102) de la revendication 1, dans lequel la partie non conductrice (206, 306, 406) comprend un matériau non conforme et non conducteur.
